(19) Europäisches Patentamt
European Patent Office
Office européen des brevets

(11) Publication number: **0 268 218 B1**

(12) **EUROPEAN PATENT SPECIFICATION**

(45) Date of publication of patent specification: **06.05.92**

(51) Int. Cl.⁵: **A61K 47/00**, A61K 47/06

(21) Application number: **87116753.2**

(22) Date of filing: **13.11.87**

# (54) Penetration enhancers for transdermal delivery of systemic agents.

(30) Priority: **19.11.86 US 932708**

(43) Date of publication of application:
**25.05.88 Bulletin 88/21**

(45) Publication of the grant of the patent:
**06.05.92 Bulletin 92/19**

(84) Designated Contracting States:
**AT BE CH DE ES FR GB GR IT LI LU NL SE**

(56) References cited:

| | |
|---|---|
| **EP-A- 0 129 284** | **EP-A- 0 164 998** |
| **WO-A-87/04594** | **FR-A- 2 152 456** |
| **FR-B- 1 243 632** | **GB-A- 1 553 310** |

**CHEMICAL ABSTRACTS, vol. 88, no. 23, 5th June 1978, page 543, abstract no. 169612d, Columbus, Ohio, US; & SU-A-528 848 (S. ORDZHONIKIDZE etc.) 05-11-1977**

**CHEMICAL ABSTRACTS, vol. 106, no. 9, 2nd March 1987, page 592, abstract no. 67103m, Columbus, Ohio, US; & ES-A-549 688 (LAZLO INTERNACIONAL S.A.) 16-03-1986**

(73) Proprietor: **WHITBY RESEARCH, Inc.**
**2801 Reserve Street**
**Richmond, VA 23227(US)**

(72) Inventor: **Minaskanian, Gevork**
**8 Havenwood**
**Irvine California 92715(US)**
Inventor: **Peck, James V.**
**2524 Bowdoin Place**
**Costa Mesa California 92626(US)**

(74) Representative: **KOHLER SCHMID + PARTNER**
**Patentanwälte**
**Ruppmannstrasse 27**
**W-7000 Stuttgart 80(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.08/2.18/2.0)

## Description

The invention generally relates to an improved method of drug delivery. More particularly, the invention relates to an improved membrane penetration enhancer for use in the transdermal delivery of systemically active drugs to humans and animals.

For some years, pharmaceutical researchers have sought an effective means of introducing drugs into the bloodstream by applying them to unbroken skin. Among other advantages, such administration can provide a comfortable, convenient, and safe way of giving many drugs now taken orally or infused into veins or injected intramuscularly.

Using skin as the portal for drug entry offers unique potential, because transdermal delivery permits close control over drug absorption. For example, it avoids factors that can cause unpredictable absorption from the gastrointestinal tract, including: changes in acidity, motility, and food content. It also avoids initial metabolism of the drug by the liver. Thus, controlled drug entry through skin can achieve a high degree of control over blood concentrations of drug.

Close control over drug concentrations in blood can translate readily into safer and more comfortable treatment. When a drug's adverse effects occur at higher concentrations than its beneficial ones, rate control can maintain the concentrations that evoke only--or principally the drug's desired actions. This ability to lessen undesired drug actions can greatly reduce the toxicity hazards that now restrict or prevent the use of many valuable agents.

Transdermal delivery particularly benefits patients with chronic disease. Many such patients have difficulty following regimens requiring several doses daily of medications that repeatedly cause unpleasant symptoms. They find the same drugs much more acceptable when administered in transdermal systems that require application infrequently--in some cases, only once or twice weekly--and that reduce adverse effects.

Transdermal delivery is feasible for drugs effective in amounts that can pass through the skin area and that are substantially free of localized irritating or allergic effects. While these limitations may exclude some agents, many others remain eligible for transdermal delivery. Moreover, their numbers will expand as pharmaceutical agents of greater potency are developed. Particularly suitable for transdermal delivery are potent drugs with only a narrow spread between their toxic and safe blood concentrations, those having gastrointestinal absorption problems, or those requiring frequent dosing in oral or injectable form.

Transdermal therapy permits much wider use of natural substances such as hormones. Often the survival times of these substances in the body are so short that they would have to be taken many times daily in ordinary dosage forms. Continuous transdermal delivery provides a practical way of giving them and one that can mimic the body's own patterns of secretion.

At present, controlled transdermal therapy appears feasible for many drugs used for a wide variety of ailments including, but not limited to, circulatory problems, hormone deficiency, respiratory ailments, and pain relief.

Percutaneous administration can have the advantage of permitting continuous adminstration of drug to the circulation over a prolonged period of time to obtain a uniform delivery rate and blood level of drug. Commencement and termination of drug therapy are initiated by the application and removal of the dosing devices from the skin. Uncertainties of administration through the gastrointestinal tract and the inconvenience of administration by injection are eliminated. Since a high concentration of drug never enters the body, problems of pulse entry are overcome and metabolic half-life is not a factor of controlling importance.

U.S. Patent Nos. 3,989,815; 3,989,816; 3,991,203; 4,122,170; 4,316,893; 4,415,563; 4,423,040; 4,424,210; and 4,444,762 generally describe a method for enhancing the topical (as contrasted to the systemic) administration of physiologically active agents by combining such an agent with an effective amount of penetration enhancer and applying the combination topically to humans or animals, in the form of creams, lotions, gels, etc.

Penetration enhancers for enhancing systemic administration of therapeutic agents transdermally are cited in U.S. Patent Nos. 4,405,616; 4,562,075; 4,031,894, 3,996,934; and 3,921,636 and in EP-A-0 129 284 and EP-A-0 164 998 and in WO-A-87/01935.

It has been discovered that the penetration enhancers previously disclosed in WO-A-87104594, which was published after the priority date enjoyed by this Patent to enhance topical delivery of physiologically active agents also enhance the transdermal delivery of systemically active agents through the skin or other body membranes of humans and animals directly into the bloodstream.

The invention therefore provides compositions for use in a method for topically administering systemically active agents through the skin or mucosal membranes of humans and animals, utilizing a transdermal device or formulation, which comprise a systemic agent and an effective amount of a membrane penetration enhancer having the structural formula

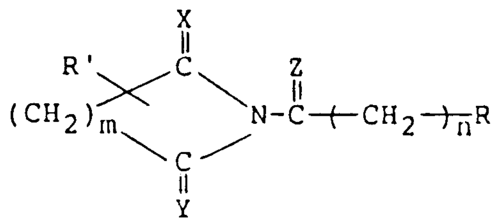

wherein each X, Y and Z may represent oxygen, sulfur or two hydrogen atoms, provided however that, when Z represents two hydrogen atoms, both X and Y represent oxygen or sulfur and when Z represents oxygen or sulfur at least one of X and Y must represent oxygen or sulfur; m is 2-6; R′ is H or a lower alkyl group having 1-4 carbon atoms; n is 0-16 and R is $-CH_3$,

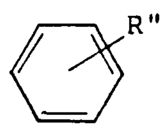

or

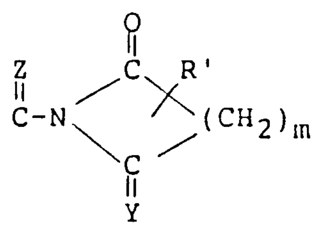

wherein R″ is H or halogen. The systemic agent being selected from haloperidol, isosorbide, dinitrate, nitroglycerine, estradiol, cloridine, propanolol, indomethacin, nifedipine, nicardipine, diclorofenac and metaprotenol.

The compositions can also be used in an improved method for administering systemically active therapeutic agents topically through the skin of humans in a transdermal device or formulation to obtain therapeutic blood levels of the therapeutic agent, wherein the improvement in said method comprises the use of an effective skin penetration enhancing amount of the above membrane penetration enhancer, with said therapeutic agent.

Preferably R is $-CH_3$ and R′ is H.

In a more preferred embodiment of the present invention R is $-CH_3$, R′ is H and m equals 4. Even more preferably, n is 4-17, e.g. 10.

The compounds useful as membrane penetration-enhancers in the formulations or devices of the instant invention may be made as described in WO-A-87/04594. Topical examples of compounds represented by the above structural formula include:

1-n-dodecylazacycloheptan-2,7-dione
1-(1-oxododecyl)azacycloheptan-2-one
1-(1-oxooctadecyl)azacycloheptan-2-one
1-(1-oxotetradecyl)azacycloheptan-2-one
1-(1-oxodecyl)azacycloheptan-2-one
1-(1-oxoundecyl)azacycloheptan-2-one
1-(1-oxotridecyl)azacycloheptan-2-one
2,2′-sebaccylibisazacycloheptan-2-one
1-n-hexanoylazacyclooctan-2-one
1,1-adipoylbisazacycloheptan-2-one
1,1′-adipoylbisazacyclopentan-2-one
1-(1-oxobutyl)azacycloheptan-2-one
1-(1-oxoheptyl)azacycloheptan-2-one
1-(1-oxopentyl)azacycloheptan-2-one
1-(1-oxohexyl)azacycloheptan-2-one
1-(1-oxooctyl)azacycloheptan-2-one

1-(1-oxononyl)azacycloheptan-2-one
1,1′-azelaoylbisazacycloheptan-2-one
1,1′-succinylbisazacycloheptan-2-one
1,1′-suberoylbisazacycloheptan-2-one
1,1′-pimeloylbisazacycloheptan-2-one
1-(1-oxobutyl)azacyclooctan-2-one
1-(1-oxopentyl)azacyclohexan-2-one
1-(1-oxobutyl)azacyclopentan-2-one
1-(1-oxoundecyl)azacyclopentan-2-one
1-(1-oxodecyl)azacyclopentan-2-one
1-(1-oxooctyl)azacyclopentan-2-one
1-(1-ocooctyl)azacyclohexan-2-one
1-(1-oxododecyl)azacyclopentan-2-one
1-(1-oxooctadecyl)azacyclohexan-2-one
1-(1-oxohexyl)azacyclohexan-2-one
1-(1-oxobutyl)azacyclohexan-2-one
1-(1-oxopentyl)azacyclopentan-2-one
1-(1-oxohexyl)azacyclopentan-2-one

Dosage forms for application to the skin or other membranes of humans and animals include creams, lotions, gels, ointments, suppositories, sprays, aerosols, buccal and sub-lingual tablets and any one of a varietary of transdermal devices for use in the continuous administration of systemically active drugs by absorption through the skin, oral mucosa or other membranes, see, for example, one or more of U.S. Patents Nos. 3,598,122; 3,598,123; 3,731,683; 3,742,951; 3,814,097, 3,921,636; 3,972,995; 3,993,072; 3,993,073; 3,996,934; 4,031,894; 4,060,084; 4,069,307; 4,201,211; 4,230,105; 4,292,299 and 4,292,303. U.S. Patent No. 4,077,407 and the foregoing patents also disclose a variety of specific systemically active agents which may also be useful in transdermal delivery.

Typical inert carriers which may be included in the foregoing dosage forms include conventional formulating materials, such as, for example, water, isopropyl alcohol, gaseous fluorocarbons, ethyl alcohol, polyvinyl pyrrolidone, propylene glycol, fragrances, gel-producing materials such as "Carbopol®", stearyl alcohol, stearic acid, spermacetic, sorbitan monooleate, "Polysorbates", "Tweens®", sorbital, methylcellulose, etc.

Systemically active agents are used in amounts calculated to achieve and maintain therapeutic blood levels in a human or animal over the period of time desired. These amounts vary with the potency of each systemically active substance, the amount required for the desired therapeutic or other effect, the rate of elimination or breakdown of the substance by the body once it has entered the bloodstream, and the amount of penetration enhancer in the formulation. In accordance with conventional prudent formulating practices, a dosage near the lower end of the useful range of a particular agent is usually employed initially and the dosage increased or decreased as indicated from the observed response, as in the routine procedure of the physician.

The amount of penetration enhancer which may be used in the invention varies from about 1 to 100 percent although adequate enhancement of penetration is generally found to occur in the range of 1 to 10 percent by weight of the formulation to be delivered. The penetration enhancer disclosed herein may be used in combination with the active agent or may be used separately as a pre-treatment of the skin or other body membrane through which the systemically-active agent is intended to be delivered.

The invention is further illustrated by the following examples which are illustrative of a specific mode of practicing the invention and is not intended as limiting the scope of the appended claims.

EXAMPLE 1

A composition in the form of a gel, suitable for transdermal delivery of haloperidol, an antidyskinetic or antipsychotic drug, is prepared by mixing the following components in the given concentrations.

| Component | Weight % |
|---|---|
| Haloperidol | 1-5 |
| 1-n-Dodecanoylazacycloheptan-2-one | 1-10 |
| Carbopol 934 P (Available from B.F. Goodrich) | 0.5-2 |
| Neutralizing Agent (NaOH) | q.s. |
| Tween-20® (Available from Atlas Chemical, a Div. of I.C.I.) | 1-10 |
| Preservative (Sorbic Acid) | q.s. |
| Antioxidant (Ascorbic Acid) | q.s. |
| Chelating Agent (Disodium salt of ethylenediaminetetraacetic acid) | q.s. |
| Deionized Water | q.s. to 100 |

This composition is topically applied to the skin of a human subject and after the passage of a suitable period of time haloperidol is found in the bloodstream of said subject.

EXAMPLE 2

When an amine, e.g. triethylamine or triethanolamine, is substituted for NaOH the results are substantially similar, i.e. a topical composition suitable for transdermally delivering haloperidol to the bloodstream is obtained.

EXAMPLE 3

When potassium sorbate, or a lower alkyl paraben, e.g. methyl, ethyl, propyl or butyl paraben are substituted for the preservative of the composition of EXAMPLE 1, the results are substantially similar, i.e. a topical composition suitble for the trnasdermal delivery of haloperidol to the bloodstream is obtained.

EXAMPLE 4

When ascorbyl palmitate, Vitamin E, thioglycerol, thioglycolic acid, sodium formaldehyde sulfoxylate, BHA, BHT, propyl gallate or sodium metabisulfite are substituted for the antioxidant of the composition formulated in EXAMPLE 1, the results are substantially similar in that a topical composition suitble for the transdermally delivering haloperidol to the bloodstream is obtained.

EXAMPLE 5

The composition of EXAMPLE 1 is prepared in the form of a sodium alginate gel by mixing the following components in the following given concentrations:

| Component | Weight % |
|---|---|
| Haloperidol | 1-5 |
| 1-Dodecanoylazacycloheptan-2-one | 1-10 |
| Sodium Alginate | 0.5-5 |
| Calcium Salts | q.s. |
| Tween-20® | 1-10 |
| Preservative* | q.s. |
| Antioxidant** | q.s. |
| Chelating Agent*** | q.s. |
| Deionized Water | to 100 |

*Suitable preservatives are those used in EXAMPLE 3 as well as sorbic acid.
**Suitabler antioxidants are those used in EXAMPLE 4 including sorbic acid.
***The chelating agent is the disodium salt of ethylenediaminetetraacetic acid.

The composition when applied topically is found to transdermally delivery haloperidol to the bloodstream of a subject.

EXAMPLE 6

The composition of EXAMPLE 1 is prepared in the form of a hydrophilic cream by mixing the following components:

| Component | |
|---|---|
| Oil Phase | Weight % |
| Cetyl Alcohol | 5-15 |
| Stearyl Alcohol | 1-5 |
| 1-n-Dodecanoylazacycloheptan-2-one | 0.5-10 |
| Glycerol Monostearate | 2-7 |
| Water Phase | |
| Sodium Laurylsulfate | 0.1 |
| Solvent* | 2-20 |
| Tween-20® | 1-5 |
| Water | q.s. to 100 |

*Suitable solvents are propylene glycol, glycerin, alcohols, for example, ethyl alcohol, isopropyl alcohol, and polyethylene glycols.

The oil phase and the water phase is made up separately, and then agitated to form an emulsion. (When, as in EXAMPLE 8, the active ingredient, is other than haloperidol, depending on its lipophilicity, it will be distributed in the oil or water phase.) This hydrophilic cream, when applied topically to the skin of a human, is found to transdermally deliver haloperidol into the bloodstream.

EXAMPLE 7

The composition of the instant invention may also be delivered by use of a polymeric matrix. For example, a solid polymer such as cellulose triacetate, polyvinyl acetate, terpolymers and copolymers of vinyl chloride and vinyl acetate, copolymers of polyvinyl alcohol and polyvinyl acetate, and silicon elaastomers is imbibed with a liquid having the following components in the given concentrations.

| Component | Weight % |
|---|---|
| Polymer | 5-40 |
| Haloperidol | q.s. |
| 1-n-Dodecanoylazacycloheptan-2-one | 0.5-80 |
| Solvent* | 5-90 |
| Surfactant** | 1-10 |
| Preservative*** | q.s. |
| Antioxidant**** | q.s. |

*Solvents may be the solvents used in EXAMPLE 6 above.
**The Surfactant may be Tween-20®, glycerol monostearate or sodium laurylsulfate.
***The preservative may be of any of the preservatives used in EXAMPLE 3 above.
****The antioxidants may be any of those used in EXAMPLE 4 above.

When solid matrix, containing the active ingredients formulated above, is contacted with the skin of a human subject, after a period of time the active agent is found in the bloodstream of said subject.

EXAMPLE 8

EXAMPLES 1 to 7 are repeated except that the following active ingredients in the given concentrations are substituted for haloperidol:

| Active Ingredient | Weight % |
|---|---|
| Isosorbide Dinitrate | 5-15 |
| Nitroglycerin | 1-5 |
| Estradiol | 1-5 |
| Clonidine | 0.5-3 |
| Propranolol | 1-5 |
| Indomethacine | 5-15 |
| Nifedipine | 1-5 |
| Nicardipine | 1-5 |
| Diclorofenac | 5-15 |
| Metaproterenol | 1-5 |

Similar results are obtained in that the active ingredient is transdermally delivered to the bloodstream of an animal.

EXAMPLE 9

EXAMPLES 1 to 8 are repeated except tha the compounds exemplified on page 6 (except for 1-n-Dodecanoylazacycloheptan-2-one) are substituted for 1-n-Dodecanoylazacycloheptan-2-one. Similar results are obtained in that the active ingredients are transdermally delivered to the bloodstream of an animal.

**Claims**

**Claims for the following Contracting States : BE, CH, DE, FR, GB, IT, LI, LU, NL, SE**

**1.** A composition for enhancing the membrane penetration of at least one topically administered systemically active agent selected from the group consisting of haloperidol, isosorbide dinitrate, nitroglycerin, estradiol, clonidine, propanolol, indomethacin, nifedipine, nicardipine, diclorofenac and metaproterenol, through the skin or mucosal membranes of humans and animals in a transdermal device or formulation, by mixing an effective amount of the membrane penetration enhancer having the structural formula

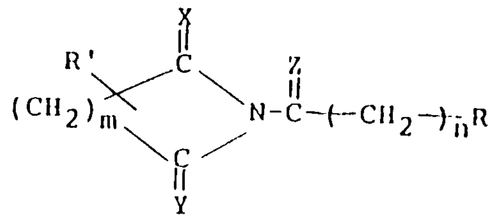

wherein each X, Y and Z may represent oxygen, sulfur or two hydrogen atoms, provided that, when Z represents two hydrogen atoms, both X and Y represent oxygen or sulfur and when Z represents oxygen or sulfur at least one of X and Y must represent oxygen or sulfur; m is 2-6; R' is H or a lower alkyl group having 1-4 carbon atoms; n is 0-16 and R is $-CH_3$,

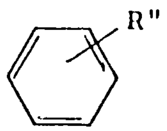

or

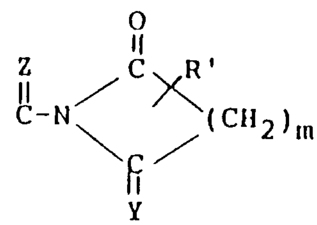

wherein R" is H or halogen,
with at least one of said active agent.

2. The composition of claim 1 wherein R' is hydrogen, m is 4, n is 4-17 and R is $CH_3$.

3. The composition of claim 2 wherein X represents two hydrogen atoms and Y and Z are oxygen.

4. The composition of claim 3 wherein n is 10.

5. The composition of claim 1 wherein the membrane penetration enhancer is 1-n-Dodecanoylazacycloheptan-2-one.

**Claims for the following Contracting States : AT, ES, GR**

1. A process for preparing a composition for enhancing the membrane penetration of at least one topically administered systemically active agent selected from the group consisting of haloperidol, isosorbide dinitrate, nitroglycerin, estradiol, clonidine, propanolol, indomethacin, nifedipine, nicardipine, diclorofenac and metaproterenol, through the skin or mucosal membranes of humans and animals in a transdermal device or formulation, by mixing an effective amount of the membrane penetration enhancer having the structural formula

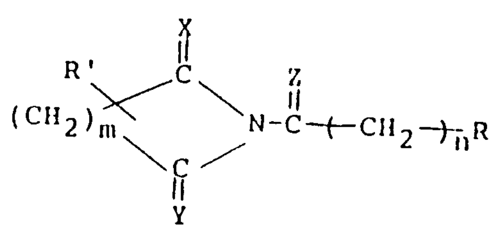

wherein each X, Y and Z may represent oxygen, sulfur or two hydrogen atoms, provided that, when Z represents two hydrogen atoms, both X and Y represent oxygen or sulfur and when Z represents oxygen or sulfur at least one of X and Y must represent oxygen or sulfur; m is 2-6; R' is H or a lower

alkyl group having 1-4 carbon atoms; n is 0-16 and R is $-CH_3$,

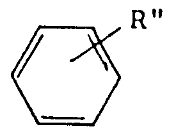

or

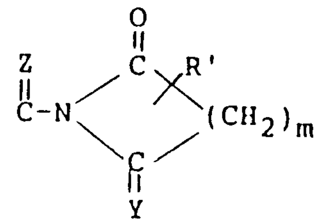

wherein R" is H or halogen,
with at least one of said active agent.

**2.** A process according to claim 1 wherein R' is hydrogen, m is 4, n is 4-17 and R is $CH_3$.

**3.** A process according to claim 2 wherein X represents two hydrogen atoms and Y and Z are oxygen.

**4.** A process according to claim 3 wherein n is 10.

**5.** A process according to claim 1 wherein the membrane penetration enhancer is 1-n-Dodecanoylazacycloheptan-2-one.

## Revendications

**Revendications pour les Etats contractants suivants : BE, CH, DE, FR, GB, IT, LI, LU, NL, SE**

**1.** Composition pour améliorer la pénétration membraneuse d'au moins un agent à action systémique administré topiquement choisi dans le groupe comprenant l'halopéridol, le dinitrate d'isosorbide, la nitroglycérine, l'oestradiol, la clonidine, le propanolol, l'indométhacine, la nifédipine, la nicardipine, le diclorofénac et le métaprotérénol, à travers la peau ou les muqueuses de l'être humain et des animaux dans un dispositif ou une formulation transdermique, en mélangeant une quantité efficace de l'agent améliorant la pénétration membraneuse répondant à la formule structurale :

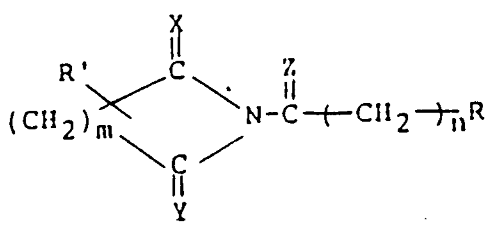

dans laquelle X, Y et Z peuvent représenter chacun de l'oxygène, du soufre ou deux atomes d'hydrogène, pour autant que, lorsque Z représente deux atomes d'hydrogène, X et Y représentent tous deux de l'oxygène ou du soufre et lorsque Z représente de l'oxygène ou du soufre, au moins l'un des X et Y doit représenter de l'oxygène ou du soufre, m est égal à 2-6, R' représente H ou un groupe

alkyle inférieur comportant 1-4 atomes de carbone, n est égal à 0-16 et R représente $-CH_3$,

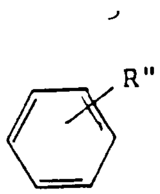

ou

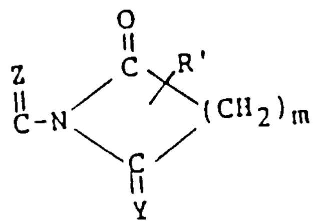

où R" représente H ou un halogène, avec au moins l'un des agents actifs précités.

2. Composition suivant la revendication 1, caractérisée en ce que R' représente de l'hydrogène, m est égal à 4, n est égal à 4-17 et R représente $CH_3$.

3. Composition suivant la revendication 2, caractérisée en ce que X représente deux atomes d'hydrogène et X et Z sont de l'oxygène.

4. Composition suivant la revendication 3, caractérisée en ce que n est égal à 10.

5. Composition suivant la revendication 1, caractérisée en ce que l'agent améliorant la pénétration membraneuse est la 1-n-dodécanoylazacycloheptan-2-one.

**Revendications pour les Etats contractants suivants : AT, ES, GR**

1. Procédé de préparation d'une composition pour améliorer la pénétration membraneuse d'au moins un agent à action systémique administré topiquement choisi dans le groupe comprenant l'halopéridol, le dinitrate d'isosorbide, la nitroglycérine, l'oestradiol, la clonidine, le propanolol, l'indométhacine, la nifédipine, la nicardipine, le diclorofénac et le métaprotérénol, à travers la peau ou les membranes muqueuses de l'être humain et des animaux dans un dispositif ou une formulation transdermique, en mélangeant une quantité efficace de l'agent améliorant la pénétration membraneuse répondant à la formule structurale :

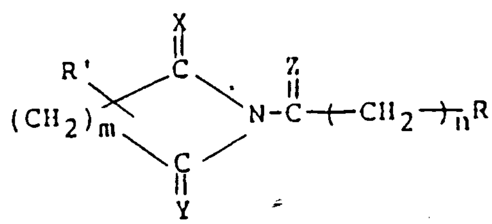

dans laquelle X, Y et Z peuvent représenter chacun de l'oxygène, du soufre ou deux atomes d'hydrogène, pour autant que, lorsque z représente deux atomes d'hydrogène, X et Y représentent tous deux de l'oxygène ou du soufre et lorsque Z représente de l'oxygène ou du soufre, au moins l'un des X et Y doit représenter de l'oxygène ou du soufre, m est égal à 2-6, R' représente H ou un groupe

alkyle inférieur comportant 1-4 atomes de carbone, n est égal à 0-16 et R représente $-CH_3$,

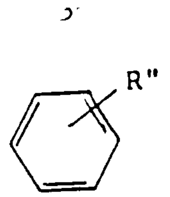

ou

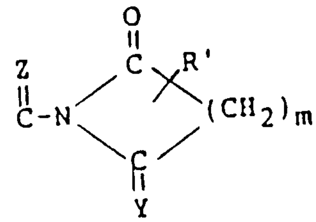

où R" représente H ou un halogène, avec au moins l'un des agents actifs précités.

**2.** Procédé suivant la revendication 1, caractérisé en ce que R' représente de l'hydrogène, m est égal à 4, n est égal à 4-17 et R représente $CH_3$.

**3.** Procédé suivant la revendication 2, caractérisé en ce que X représente deux atomes d'hydrogène et X et Z sont de l'oxygène.

**4.** Procédé suivant la revendication 3, caractérisé en ce que n est égal à 10.

**5.** Procédé suivant la revendication 1, caractérisé en ce que l'agent améliorant la pénétration membraneuse est la 1-n-dodécanoylazacycloheptan-2-one.

## Patentansprüche

### Patentansprüche für folgende Vertragsstaaten : BE, CH, DE, FR, GB, IT, LI, LU, NL, SE

**1.** Zusammensetzung zur Erhöhung der Membrandurchdringung von mindestens einem topisch verabreichten systemischen Wirkstoff ausgewählt aus der Gruppe bestehend aus Haloperidol, Isosorbiddinitrat, Nitroglycerin, Estradiol, Clonidin, Propanolol, Indomethacin, Nifedipin, Nicardipin, Dichlorfenac und Metaproterenol durch die Haut oder mukösen Membranen von Mensch und Tier in einer transdermalen Vorrichtung oder Zubereitung, bei der man eine wirksame Menge einer die Membrandurchdringung erhöhenden Substanz mit der Strukturformel

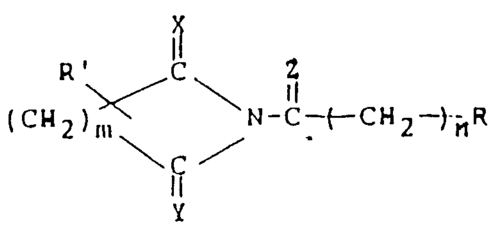

in der X, Y und Z jeweils Sauerstoff, Schwefel oder zwei Wasserstoffatome bedeuten, mit der Maßgabe, daß, wenn Z2 Wasserstoffatome bedeutet sowohl X und Y Sauerstoff oder Schwefel bedeuten und wenn Z Sauerstoff oder Schwefel bedeutet mindestens eines von X und Y Sauerstoff oder Schwefel bedeutet; m 2 bis 6 ist; R' H oder $C_{1-4}$-Niedrigalkylgruppe bedeutet; n 0 bis 16 ist und R $CH_3$,

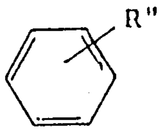

oder

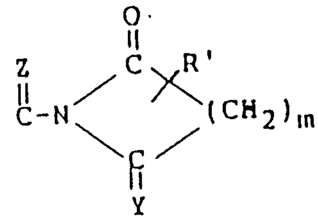

bedeutet,
worin R'' Wasserstoff oder Halogen ist,
mit mindestens einem der genannten Wirkstoffe vermischt.

**2.** Zusammensetzung nach Anspruch 1, in der R' Wasserstoff ist, m 4 ist, n 4 bis 17 ist und R $CH_3$ ist.

**3.** Zusammensetzung nach Anspruch 2, in der X 2 Wasserstoffatome bedeutet und Y und Z Sauerstoff sind.

**4.** Zusammensetzung nach Anspruch 3, in der n 10 ist.

**5.** Zusammensetzung nach Anspruch 1, in der die Membrandurchdringung erhöhende Substanz 1-n-Dodecanoylazacycloheptan-2-on ist.

**Patentansprüche für folgende Vertragsstaaten : AT, ES, GR**

**1.** Verfahren zur Herstellung einer Zusammensetzung zur Erhöhung der Membrandurchdringung von mindestens einem topisch verabreichten systemischen Wirkstoff ausgewählt aus der Gruppe bestehend aus Haloperidol, Isosorbiddinitrat, Nitroglycerin, Estradiol, Clonidin, Propanonol, Indomethacin, Nifedipin, Nicardipin, Dichlorfenac und Metaproterenol durch die Haut oder mukösen Membranen von Mensch und Tier in einer transdermalen Vorrichtung oder Zubereitung, bei dem man eine wirksame Menge einer die Membrandurchdringung erhöhenden Substanz mit der Strukturformel

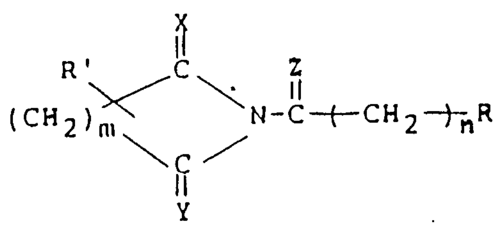

worin X, Y und Z jeweils Sauerstoff, Schwefel oder zwei Wasserstoffatome bedeuten, mit der Maßgabe, daß, wenn Z 2 Wasserstoffatome bedeutet sowohl X als auch Y Sauerstoff oder Schwefel bedeuten und wenn Z Sauerstoff oder Schwefel bedeutet, mindestens eines von X und Y Sauerstoff oder Schwefel bedeutet; m 2 bis 6 ist; R' H oder eine $C_{1-4}$-Niedrigalkylgruppe bedeutet; n 0 bis 16 ist und R $CH_3$,

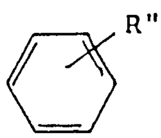

oder

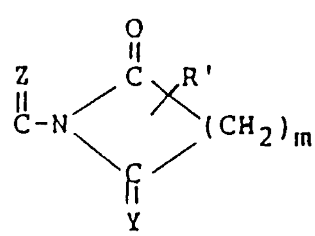

bedeutet,
worin R'' Wasserstoff oder Halogen ist,
mit mindestens einem der genannten Wirkstoffe vermischt.

**2.** Verfahren nach Anspruch 1, bei dem R' Wasserstoff ist, m 4 ist, n 4 bis 17 ist und R $CH_3$ ist.

**3.** Verfahren nach Anspruch 2, bei dem X 2 Wasserstoffatome bedeutet und Y und Z Sauerstoff sind.

**4.** Verfahren nach Anspruch 3, bei dem n 10 ist.

**5.** Verfahren nach Anspruch 1, bei dem die Membrandurchdringung erhöhende Substanz 1-n-Dodecanoylazacycloheptan-2-on ist.